# EUROPEAN PATENT SPECIFICATION

(11) **EP 1 689 338 B1**
(45) Date of publication and mention of the grant of the patent: **16.01.2013**
(21) Application number: 04819550.7
(22) Date of filing: 18.11.2004
(51) Int. Cl.: A61L 15/46, A61L 15/58

(54) **ANTIMICROBIAL ADHESIVE SYSTEM**
ANTIMIKROBIELLES KLEBESYSTEM
SYSTEME ADHESIF ANTIMICROBIEN

(30) Priority: 19.11.2003 US 717380
(43) Date of publication of application: 16.08.2006
(73) Proprietor: MEDICAL CONCEPTS DEVELOPMENT, INC., Woodbury, Minnesota 55125 (US)
(72) Inventor: COX, David, D., Woodbury, Minnesota 55125 (US); LUND, Robert, E., Eagan, Minnesota 55122 (US); ANNETT, Leland, W., Baytown, Minnesota 55082 (US)
(74) Representative: Kirschner, Klaus Dieter
(86) International application number: PCT/US2004/038779
(87) International publication number: WO 2005/051438

(56) References cited:
- EP-A- 0 361 722
- US-A- 5 829 442

## Description

The present invention relates to a medical grade, antimicrobial-containing adhesive particularity suited for use in skin contact applications, such as with surgical drapes, tapes and wound dressings. It is recognized that numerous pathogens are present on human skin. Therefore, in a hospital environment, it is generally desired that the growth of disease-producing microorganisms be inhibited, and preferably that these microorganisms be destroyed so as to control patient infection and encourage wound healing. Under most circumstances, the bacteria of normal skin cannot cause wound infections, but in the presence of foreign materials or open wounds, the pathogenic potential of these bacteria appears to be considerably enhanced. Furthermore, the likelihood of bacterial contamination is at a peak immediately preceding, during, and following surgical procedures. Accordingly, to prevent contamination, it is imperative that the skin be effectively disinfected before a surgical incision is made and during the entire surgical procedure.

In response to such concerns, many topical antimicrobial agents have been developed. These agents typically are in the form of preoperative skin preps, surgical scrub tissues, washes, wound cleaners, lotions and ointments. A recognized limitation to such topical applications are a short effective delivery time.

Microorganisms that may have survived the initial application of such a topical antimicrobial agent can act as a seed, causing the pathogen population in some instances to regenerate or rise to their initial levels. Thus, continuous application of an antimicrobial agent to the site is recognized as a means of inhibiting this increase in population.

It has been recognized that a continuous or longer lasting antimicrobial effect may be achieved by incorporating the antimicrobial agent into an adhesive layer or into a surgical incise drape material itself.

Berglund et al. (US-A- 4,310, 509) disclose that it is known to incorporate biologically active agents into adhesive layers on a substrate to provide continuous application of such agent to the body. Disclosed examples of known adhesives containing antimicrobial agents include US-A- 2,137, 169, wherein phenol, thymol, methanol, etc. are added to a starch adhesive; US-A- 3,249, 109 where benzocaine was added to a tacky gelatin; US-A- 3,632, 740 where a corticosteroid is added to an adhesive; US-A-3,734, 097 where a microencapsulated anti- neoplastic agent is added to an adhesive ; US-A- 4,073, 291 where Tretinoin is added to an adhesive; US-A- 3,769, 071 where 5-fluorouracil is incorporated into an adhesive ; and US-A- 3,896, 789 where retinoic acid is incorporated into a pressure-sensitive adhesive tape. Berglund et al. further teach that the prior art attempts to include an antimicrobial agent in an adhesive did not include the use of a broad spectrum antimicrobial because such adhesives had been frustrated by uncontrollable release of the agent with accompanying skin irritation in some patients, along with failure to obtain sufficient antimicrobial activity.

Berglund et al. disclose a pressure sensitive adhesive composition which contains chlorhexidene, polyvinylpyrrolidone iodine or iodine which is applied onto a polymer sheet material, such as polyethylene or polyurethane, for use as a surgical drape. The disclosed drape is applied to the skin with the adhesive side contacting the skin so that the antimicrobial agent can be released from the adhesive to the wound area prior to and during incision. The process for making the adhesive disclosed by Berglund et al. involves forming an emulsifiable concentrate or an organic solution concentrate of a broad spectrum antimicrobial agent and mixing it into an adhesive, such that the broad spectrum antimicrobial is homogeneously dispersed as a separate phase throughout the adhesive medium. The homogenous dispersion is then spread or coated to a substantially uniform layer followed by drying of the wet layer in order to remove the solvents.

Rosso et al. (US-A- 4,323, 557) disclose a drape incorporating a pressure sensitive adhesive utilizing n-vinylpyrrolidione residues in the polymer backbone. Iodine is complexed with these residues to provide an antimicrobial effect. Rosso et al. espouse the stability of the adhesive composition over the prior art compositions.
The process for forming the adhesive composition disclosed by Rosso et al. involves forming a pressure-sensitive adhesive and mixing into it an antimicrobial treating solution comprising iodine, an iodide, and a solvent. The resulting composition preferably contains n-vinylpyrrolidone in the backbone of the pressure-sensitive adhesive which serves to complex the iodine. Rosso et al. disclose that the composition may be either attached directly onto a flexible backing substrate or formed onto a release liner for later use. Once applied, the solvents are then evaporated by means known to the art, whereby an adhesive film is formed which is useable in or on tapes, drapes and other medical devices.

Mixon et al. (US-A- 5, 069, 907) disclose a surgical drape having incorporated therein a broad spectrum antimicrobial agent. The drape comprises a synthetic polymeric film or fabric having incorporated therethrough an amount of antimicrobial agent. The drape may optionally have an adhesive layer attached to one of its external surfaces, wherein the adhesive layer can have dispersed therethrough an antimicrobial agent. The preferred antimicrobial agent used is 5-chloro-2- (2, 4-dichlorophenoxy) phenol. Suitable adhesives utilized include polyacrylate adhesives.

Mixon et al. disclose a large number of antimicrobial agents which were contemplated for use with the disclosed composition. These include metal salts, typical antibiotics, antibacterial agents such as chlorhexidine and its salts, quaternary ammonium compounds, iodophors such as povidone iodine, acridine compounds, biguanidine compounds, and a preferred antimicrobial agent 5- chloro-2- (2, 4-dichlorophenoxy) phenol. Mixon et al. further disclose that these same antimicrobial agents, which they propose to utilize within the polymer composition for their surgical drape, can also be utilized in an adhesive composition. Mixon et al. further state that the antimicrobial agent can be directly applied to the surgical drape in solution as an aqueous dispersion, as a hot melt, or by a transfer process using known techniques, such as knife, roller-coating, or curtain-coating methods. The transfer process is disclosed as particularly preferred. In a transfer process, the adhesive emulsion, including water or a different solvent, optionally containing an antimicrobial agent, is spread onto a sheet of release paper and dried to remove the water or solvent. The surgical drape is then brought into contact with the adhesive and calendared to insure that the adhesive adheres to the drape. The surgical drape will then generally include a release sheet covering the adhesive, and the release sheet on which the adhesive is deposited can be used for that purpose, or that release sheet can be removed and replaced with another release sheet. In embodiments where the adhesive contains an antimicrobial agent, the mixture of adhesive and antimicrobial agent is dried after coating on the release sheet, and the antimicrobial agent remains dispersed in the adhesive.

Generally, presently known antimicrobial agents are limited in their ability to withstand heat during processing. The lack of heat stability of n-vinyl pyrrolodione iodine has limited the ability for drapes having this antimicrobial agent from being ethylene oxide sterilized under heat stress. Further, many of the antimicrobial compounds cannot be radiation sterilized. Thus, each prior art reference teaches that it is preferred to apply the antimicrobial adhesive in conjunction with a solvent followed by subsequent evaporation of the solvent.

Accordingly, the need exists for an adhesive composition having an antimicrobial agent dispersed therethrough which is heat stable, and solventless. Similarly, it is highly desirable and advantageous to provide an antimicrobial system requiring neither an emulsion of the antimicrobial agent, or the removal of excess solvent. Such composition or compositions would eliminate the need for use of solvents with their potential environmental effects and would eliminate the need for removing such solvent from the adhesive after application to the drapes. Furthermore, a particularly heat stabile formulation would allow ethylene oxide sterilization under heat stress or radiation sterilization.

EP 0361722 discloses an antibacterial pressure sensitive adhesive composition suitable for medicinal application to an infection prone skin area comprising a mixture of a solid hydrophobic acrylic adhesive, silver sulfadiazine and inter-ally crosslinked sodium carboxymethylcellulose and its method of use. The the release of silver sulfadiazine from acrylic-based adhesives in accordance with the compositions and methods of this invention is supposed to be improved. The EP 0361722, therefore, relates to an antibacterial pressure sensitive adhesive composition suitable for medicinal application to an infection prone skin area comprising a mixture of a solid hydrophobic acrylic adhesive; an antibacterial effective amount of silver sulfadiazine; and an effective amount of internally crosslinked sodium carboxymethylcellulose to release the silver sulfadiazine from the adhesive mixture to provide effective antibacterial action to the area of application.

US-A-5 829 442 discloses an adhesive composition having dispersed therein a broad spectrum antimicrobial agent for use in medical applications, such as an adhesive for surgical drapes, wound dressings and tapes. The adhesive is composed of acrylic polymers, tackifiers and a preferred antimicrobial agent, diiodomethyl-p-tolylsulfone. The adhesive composition is essentially without solvent and capable of application in a hot melt process while maintaining stability at elevated temperatures in the range of 275 °F (135°C) to 350 °F (177"C) which not only allows hot melt application, but allows for ethylene oxide sterilization under heat stress.

The objectives mentioned above are achieved by the adhesive composition according to claim 1. Preferred embodiments of the invention are characterized in the sub-claims.
An adhesive composition having dispersed therein a broad spectrum antimicrobial agent for use in medical applications, such as an adhesive for surgical drapes, wound dressings and tapes is provided.
The subject adhesive composition is formulated as a solvent based system wherein an emulsion of the antimicrobial agent and the removal of excess solvent is avoided.

Specifically, the adhesive is for skin-contact applications, for example, surgical drapes, tapes and wound dressings. The antimicrobial agent utilized is diiodomethyl-p-tolylsulfone with a preferred concentration of antimicrobial agents in the adhesive of about 0.1% to about 2% loading by weight.

The antimicrobial agent is homogeneously dispersed through the adhesive layer. Active antimicrobial molecules continually disassociate from the surface or leach out of the adhesive matrix over time, delivering biocidal activity at a distance from the adhesive surface. Applicants have conclusively demonstrated this property by zone of inhibition tests on a wide variety of infectious organisms. These tests conclusively showed that microbes were inhibited at a distance from the sample.

In a preferred application, the antimicrobial adhesive composition of the present invention is utilized to overly a polymeric substrate to form a surgical drape. The polymeric substrate is preferably a polyester or co-polyester sheet material which may have incorporated therein or coated on the side opposite the adhesive an antimicrobial agent.

The solvent based adhesive composition formulation of a preferred embodiment of the subject invention is particularly advantageous as it does not require an emulsion of the antimicrobial agent, nor the removal of excess solvent, and generally includes an acrylic polymer, and an effective amount of diiodomethyl-p-tolylsufone dispersed throughout the acrylic polymer. The acrylic polymer suitably comprises a mixture of acrylic resin solutions, more particularly, self-curing and non self-curing acrylic resin solutions, the non self-curing arylic resin solution preferably present in the mixture to a greater extent than the self-curing acrylic resin solution.

These and various other advantages and features of novelty which characterize the present invention are pointed out with particularity in the claims annexed hereto and forming a part hereof. However, for a better understanding of the invention, its advantages, and the objects obtained by its use, reference should be made to the accompanying descriptive matter in which there are illustrated and described preferred embodiments of the present invention.

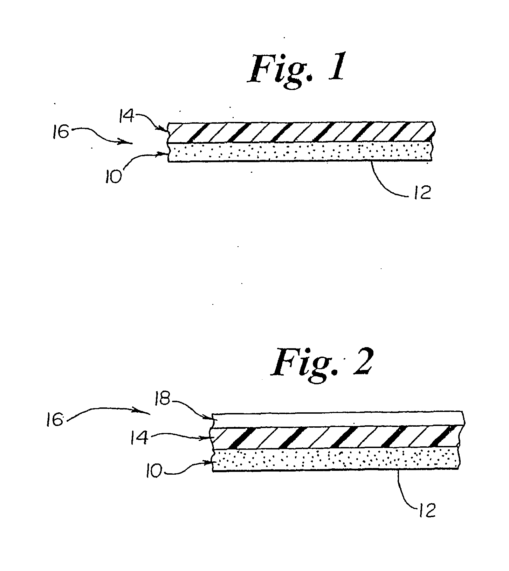

FIG. 1 is an enlarged, sectional illustration of a first embodiment of the present invention;
FIG. 2 is an enlarged, sectional illustration of a second embodiment of the present invention ;

As required, detailed embodiments of the present invention are disclosed herein. However, it is to be understood that the disclosed embodiments are merely exemplary of the present invention which may be embodied in various systems. Therefore, specific details disclosed herein are not to be interpreted as limiting, but rather as a basis for the claims and as a representative basis for teaching one skilled in the art to variously practice the present invention.

The present invention is an adhesive compound which incorporates an adhesive component together with a broad spectrum antimicrobial agent dispersed therethrough. The antimicrobial agent is homogeneously dispersed throughout the adhesive layer 10. Active antimicrobial molecules of the present composition disassociate from the surface or leach out of the adhesive matrix over time, delivering biocidal activity at a distance from the adhesive surface 12. Applicants have conclusively demonstrated by zone of inhibition tests on a wide variety of infections organisms the efficacy of the present composition. These tests showed that microbes were inhibited and killed at a distance from the sample as detailed in the attached experimental examples. In all embodiments, an adhesive composition is provided, the formulations thereof being characterized by an acrylic polymer and an effective amount of diiodomethyl-p-tolylsufone dispersed throughout the acrylic polymer.

The adhesive of the present invention is specifically suited for use in skin contact applications during and after medical procedures, for example ; as an adhesive in surgical drapes 16, wound dressings and tapes.

As earlier suggested, the form of subject antimicrobial adhesive composition or system of the subject invention is fairly characterized as being solvent based, such system not requiring a solution or concentrate of the antimicrobial agent or the drying out of the solvent following the spreading or coating on a substrate. As the disclosed antimicrobial adhesive system utilizes antimicrobial agent in the form of a solid powder, it is readily, and directly compounded into the pressure sensitive acrylic adhesive, then, spread or coated onto a substrate (e. g. , a surgical drape) without the drying requirement of the solid form of the adhesive as has been the typical and widespread practice. No additional solvent from agent is added. The subject system does not require an emulsion of the antimicrobial agent and the removal of excess solvent. Such composition formulation thereby simplifies a substrate coating process, and further allows for a more consistent, predictable, and effective result.

Further with respect to the antimicrobial agent of the subject system, the use of elemental iodine or an iodide salt in a solvent solution to provide an iodine active kill mechanism is avoided, instead the subject active antimicrobial agent is characterized by a molecule containing two iodides stabilized by the larger tolyl- phenol based group. The iodides of such molecule are harder to remove, thereby providing, among other advantages: a more heat stable and less water soluble antimicrobial agent; easier manufacture due as no special packaging and handling is required; less volatility when performing ETO sterilized ; and, reduced susceptibility to leaching of the adhesive in wet environments.

The subject antimicrobial adhesive composition or system, especially suited for skin contact applications, generally includes an acrylic polymer, and an effective amount of diiodomethyl-p-tolylsufone dispersed throughout the acrylic polymer. The acrylic polymer suitably comprises a mixture of acrylic resin solutions, more particularly, self-curing and non self-curing acrylic resin solutions, the non self-curing acrylic resin solution preferably present in the mixture to a greater extent than the self-curing acrylic resin solution.

The majority acrylic resin solution of the mixture of acrylic resin solutions of the preferred antimicrobial adhesive composition general includes ethyl acetate, toluene, and about 40-45 wt% solids, more particularly, about 83 parts by weight ethyl acetate, about 17 parts by weight toluene, and about 40-42 wt% solids. Such acrylic resin solution is commercial available under the tradename Gela@ Multipolymer Solution 788.

The minority acrylic resin solution of the mixture of acrylic resin solutions of the preferred antimicrobial adhesive composition general includes ethyl acetate, ethanol, toluene, and about 30-35 wt% solids, more particularly, about 48 parts by weight ethyl acetate, about 40 parts by weight ethanol, about 12 parts by weight toluene, and about 31-34 wt% solids. Such acrylic resin solution is commercial available under the tradename Gelva@ Multipolymer Solution 737.

The antimicrobial agent of the subject antimicrobial adhesive composition or system, as previously noted, comprises a diiodomethyl-p-tolylsulfone, e. g. , AMICAL 48, available from Angus Chemical Company. A preferred antimicrobial adhesive composition, and one subject to testing subsequently discussed, is characterized as follows:

| Wt% | Constituent |
|---|---|
| 89.67 | GelvaX solution 788 |
| 10.00 | Gela@ solution 737 |
| 0.33 | AMICAL 48 (5% in ethyl acetate) |
| 0.0245 | DC Yellow 11 (0. 3% in toluene) |
| 0.0205 | MX 643 (0. 5% in toluene) |

As to efficacy testing, an exemplary sheet sample was produced comprising a cover sheet, 3,8 mm (1.5 mils) of acrylic PSA, BIOFLEX 235- 01, and a casting sheet. The solid content of aforementioned 90%/10% solution is about 41.25% which equates to 0. 8% solids on solids of antimicrobial. The efficacy results of the subject system, namely zone of inhibition and log reduction effectiveness testing, and a discussion thereof, generally follows.

With regard to the zone of inhibition testing, the protocol associated therewith identifies the antimicrobial surfaces by placing a 6.5 mm disk of the test material on agar seeded with one cultured organism incubated and evaluated for the size (diameter) under the disk free of organisms. This zone may extend beyond the size of the disk depending on the level of antimicrobial activity. Antibiotics and skin prep solutions will have much larger zones than antimicrobial adhesives due to the high available concentration of the antimicrobial agent. Antimicrobial adhesives are predominantly effective on contact, and therefore are effective under the disk but not much beyond the disk's border. Three different organisms were tested with the test article, namely the sheet samples heretofore described, with 3,8 mm (1.5 mils) of the preferred solvent based antimicrobial adhesive system, gentamicin antibiotic positive control, and a plastic disk negative control.

Tables 1A & 1B summarize zone of inhibition test results for a solvent based antimicrobial adhesive solvent formulation for the subject invention.

**TABLE IA**

| **Plate** | **Test Article** | **Positive Control Article** | **Negative Control Article** |
|---|---|---|---|
| 1 | 6.5 mm | 24.0 mm | 0 mm |
| 2 | 6.5 mm | 23.5 mm | 0 mm |
| 3 | 6.5 mm | 24.0 mm | 0 mm |

**TABLE IB**

| **Plate** | | **Test Article** | **Positive Control Article** | **Negative Control Article** |
|---|---|---|---|---|
| **Plate 1** | *S. aureus* | 6.5 mm | 21.5 mm | 0 mm |
| | *E. coli* | 6.5 mm | 27.0 mm | 0 mm |
| | *P. aeroginosa* | 6.5 mm | 24.0 mm | 0 mm |
| **Plate 2.** | *S. aureus* | 7.0 mm | 23.5 mm | 0 mm |
| | *E. coli* | 6.5 mm | 27.0 mm | 0 mm |
| | *P. aeroginosa* | 6.5 mm | 23.5 mm | 0 mm |
| **Plate 3** | *S. aureus* | 6.5 mm | 21.5 mm | 0 mm |
| | *E. coli* | 6.5 mm | 25.0 mm | 0 mm |
| | *P. aeroginosa* | 6.5 mm | 24.0 mm | 0 mm |

With reference now to Tables IA & IB, the test article killed on contact under the disk with a zone of 6.5mm. The positive control showed significant kill, and the negative control showed no zone or kill under the disk. These results are consistent with the hot melt composition characterized by the subject diiodo-sulfone antimicrobial agent.

With regard to Table IA, a stock suspension of Pseudomonas Aeruginosa (AT C #9027) was diluted with sodium chloride, the concentration being adjusted to 1 x 108 CFU/ML using 0.5 McFarland standard. Zones of inhibition were measured, and are reported in millimeters.

With regard to the results of Table IB, stock suspensions of Pseudomonas Aeruginosa, Escherichia coli (ATC #8739) and Staphylococcus aureus (ATCC #6538) were diluted with the concentration adjusted to 1 x 108 CFU/ML using 0.5 McFarland standard. Zones of inhibition were measured, and are reported in millimeters.

Tables IIA-IIC summarize a first set of log reduction effectiveness test results for the solvent based antimicrobial adhesive solvent formulation as reported with respect to Tables IIIA & IIIB; and, Tables A to IIIC summarize a further or second set of log reduction effectiveness test results for the solvent based antimicrobial adhesive solvent formulation as reported with respect to Tables IIA-IIC.

**TABLE IIA**

| *Staphylococcus epidermidis* | | | | | |
|---|---|---|---|---|---|
| | **Dilution Factor** | **Plate 1** | **Plate 2** | **Mean** | **CFU/mL** |
| | 10⁴ | 6 | 8 | 7 | 7 x 10⁶ |
| | 10³ | 52 | 52 | 52 | 5.2 x 10⁶ |
| | Actual inoculum added: 2.6 x 10⁴ | | | | |

| *Escherichia coli* | | | | | |
|---|---|---|---|---|---|
| | **Dilution Factor** | **Plate 1** | **Plate 2** | **Mean** | **CFU/mL** |
| | 10⁴ | 151 | 146 | 148.5 | 1.5 x 10⁷ |
| | 10³ | TNTC | TNTC | TNTC | N/A |
| Actual inoculum added: 7.5 x 10^{b} | | | | | |

| *Enterococcus faecium* | | | | | |
|---|---|---|---|---|---|
| | **Dilution Factor** | **Plate 1** | **Plate 2** | **Mean** | **CFU/mL** |
| | 10⁴ | 251 | 273 | 262 | 2.6 x 10⁷ |
| | 10³ | TNTC | TNTC | TNTC | N/A |
| | Actual inoculum added: 1.3 x 10⁶ | | | | |

| *Streptococcus pyrogenes* | | | | | |
|---|---|---|---|---|---|
| | **Dilution Factor** | **Plate 1** | **Plate 2** | **Mean** | **CFU/mL** |
| | 10⁴ | 59 | 73 | 66 | 6.6 x 10⁶ |
| | 10³ | TNTC | TNTC | TNTC | N/A |
| | Actual inoculum added: 3.3 x 10⁵ | | | | |

| *Streptococcus aureus* | | | | | |
|---|---|---|---|---|---|
| | **Dilution Factor** | **Plate 1** | **Plate 2** | **Mean** | **CFU/mL** |
| | 10⁴ | 63 | 54 | 58.5 | 5.9 x 10⁷ |
| | 10³ | TNTC | TNTC | TNTC | N/A |

| | | | | | |
|---|---|---|---|---|---|
| Actual inoculum added: 3 x 10⁵ | | | | | |

**TABLE IIB**

| **6 Hours A0.3 *Staphylococcus epidermidis*** | | | | | | |
|---|---|---|---|---|---|---|
| **Amount Plated** | **Dilution Factor** | **Replicate 1** | **Replicate 2** | **Replicate 3** | **Mean** | **Actual CFU/Test Article** |
| **0.1 mL** | **1000** | 0 | 6 | 9 | 5 | 5x10³ |
| **1.0 mL** | **100** | 52 | 58 | 42 | 50.7 | 5.1 x 10³ |
| **10.0 mL** | **10** | TNTC | TNTC | TNTC | TNTC | TNTC |

| **6 Hours Control 2 *Staphylococcus epidermidis*** | | | | | | |
|---|---|---|---|---|---|---|
| **Amount Plated** | **Dilution Factor** | **Replicate 1** | **Replicate 2** | **Replicate 3** | **Mean** | **Actual CFU/Test Article** |
| **0.1 mL** | **1000** | 22 | 14 | 11 | 15.6 | 1.6 x 10⁴ |
| **1.0 mL** | **100** | 86 | 68 | 53 | 69 | 6.9 x 10³ |
| **10.0 mL** | **10** | TNTC | TNTC | TNTC | TNTC | TNTC |
| **6 Hours A0.3 *Escherichia coli*** | | | | | | |
| **Amount Plated** | **Dilution Factor** | **Replicate 1** | **Replicate 2** | **Replicate 3** | **Mean** | **Actual CFU/Test Article** |
| **0.1 mL** | **1000** | TNTC | TNTC | TNTC | TNTC | TNTC |
| **1.0 mL** | **100** | TNTC | TNTC | TNTC | TNTC | TNTC |
| **10.0 mL** | **10** | TNTC | TNTC | TNTC | TNTC | TNTC |
| **6 Hours Control 2 *Escherichia coli*** | | | | | | |
| **Amount Plated** | **Dilution Factor** | **Replicate 1** | **Replicate 2** | **Replicate 3** | **Mean** | **Actual CFU/Test Article** |
| **0.1 mL** | **1000** | 6 | 11 | 9 | 8.67 | 8.7 x 10³ |
| **1.0 mL** | **100** | 41 | 53 | 42 | 45.3 | 4.5 x 10³ |
| **10.0 mL** | **10** | TNTC | TNTC | TNTC | TNTC | TNTC |
| **6 Hours A0.3 *Enterococcus faecium*** | | | | | | |
| **Amount Plated** | **Dilution Factor** | **Replicate 1** | **Replicate 2** | **Replicate 3** | **Mean** | **Actual CFU/Test Article** |
| **0.1 mL** | **1000** | 78 | 42 | 138 | 86 | 8.6 x 10⁴ |
| **1.0 mL** | **100** | TNTC | TNTC | TNTC | TNTC | TNTC |
| **10,0 mL.** | **10** | TNTC . | TNTC | TNTC | TNTC | TNTC |

| **6 hours *E. faecium* A0.3** | | | | | | |
|---|---|---|---|---|---|---|
| **Amount plated** | **Dilution Factor** | **Replicate 1** | **Replicate 2** | **Replicate 3** | **Mean** | **Actual CFU/Test Article** |
| 0.01mL | 10⁴ | 43 | 39 | 30 | 37 | 3.7 x 10⁵ |
| 0.1 mL | 10³ | TNTC | TNTC | TNTC | TNTC | TNTC |
| 1.0mL | 10² | TNTC | TNTC | TNTC | TNTC | TNTC |
| 10.0 mL | 10 | TNTC | TNTC | TNTC | TNTC | TNTC |

| **6 hours *E*. *faecium* Control 2** | | | | | | |
|---|---|---|---|---|---|---|
| **Amount plated** | **Dilution Factor** | **Replicate 1** | **Replicate 2** | **Replicate 3** | **Mean** | **Actual GFU/Test Article** |
| 0.01 mL | 10⁴ | 24 | 55 | 63 | 47 | 4.7x10⁵ |
| 0.1 mL | 10^{a} | TNTC | TNTC | TNTC | TNTC | TNTC |
| 1.0 mL | 10² | TNTC | TNTC | TNTC | TNTC | TNTC |
| 10.0 mL | 10 | TNTC | TNTC | TNTC | TNTC | TNTC |

| **6 hours *E. coli* A0.3** | | | | | | |
|---|---|---|---|---|---|---|
| **Amount plated** | **Dilution Factor** | **Replicate 1** | **Replicate 2** | **Replicate 3** | **Mean** | **Actual CFU/Test Article** |
| 0.01 mL | 10⁴ | 8 | 2 | NOC | 3 | 3.0 x 10⁴ |
| 0.1 mL | 10³ | 29 | 60 | 93 | 61 | 6.1 x 10⁴ |
| 1.0 mL | 10² | 159 | TNTC | TNTC | UNTO | TNTC |
| 10.0 mL | 10 | TNTC | TNTC | TNTC | TNTC | TNTC |

| **6 hours *E*. *coli* Control 2** | | | | | | |
|---|---|---|---|---|---|---|
| **Amount plated** | **Dilution Factor** | **Replicate 1** | **Replicate 2** | **Replicate 3** | **Mean** | **Actual CFU/Test Article** |
| 0.01 mL | 10⁴ | 57 | 40 | 60 | 52 | 5.2x10⁵ |
| 0.1 mL | 10³ | TNTC | TIC | 153 | TNTC | TNTC |
| 1.0 mL | 10² | TNTC | TNTC | TNTC | TNTC | TNTC |
| 10.0 mL. | 10 | TNTC | TNTC | TNTC | TNTC | TNTC |

| **6 hours *C. albicans* A0.3** | | | | | | |
|---|---|---|---|---|---|---|
| **Amount plated** | **Dilution Factor** | **Replicate 1** | **Replicate** | **2 Replicate 3** | **Mean** | **Actual CFU/Test Article** |
| 0.01 mL | 10⁴ | NOC | NOC | NOC | NOC | NOC |
| 0.1 mL | 10³ | NOC | NOC | NOC | NOC | NOC |
| 1.0 mL | 10² | NOC | NOC | 7 | 2 | 2.0 x 10² |
| 10.0mL | 10 | 33 | 37 | 65 | 45 | 4.6 x 10² |

| **6 hours *C. albicans* Control 2** | | | | | | |
|---|---|---|---|---|---|---|
| **Amount plated** | **Dilution Factor** | **Replicate 1** | **Replicate 2** | **Replicate 3** | **Mean** | **Actual CFU/Test Article** |
| 0.01 mL | 10⁴ | NOC | NOC | NOC | NOC | NOC |
| 0.1 mL | 10³ | NOC | NOC | NOC | NOC | NOC |
| 1.0 mL | 10² | NOC | 2 | 1 | 3 | 3.0 x 10² |
| 10.0 mL | 10 | 36 | 49 | 63 | 46 | 4.6 x 10² |

**TABLE IIC**

| **6 Hours A0.3 *Staphylococcus epidermidis*** | | | | | | |
|---|---|---|---|---|---|---|
| **Amount Plated** | **Dilution Factor** | **Replicate 1** | **Replicate 2** | **Replicate 3** | **Mean** | **Actual CFU/Test Article** |
| **0.1 mL** | **1000** | 0 | 6 | 9 | 5 | 5x10³ |
| **1.0 mL** | **100** | 52 | 58 | 42 | 50.7 | 5.1 x 10³ |
| **10.0 mL** | **10** | TNTC | TNTC | TNTC | TNTC | TNTC |

| **6 Hours Control 2 *Staphylococcus epidermidis*** | | | | | | |
|---|---|---|---|---|---|---|
| **Amount Plated** | **Dilution Factor** | **Replicate 1** | **Replicate 2** | **Replicate 3** | **Mean** | **Actual CFU/Test Article** |
| **0.1 mL** | **1000** | 22 | 14 | 11 | 15.6 | 1.6 x 10⁴ |
| **1.0 mL** | **100** | 86 | 68 | 53 | 69 | 6.9 x 10³ |
| **10.0 mL** | **10** | TNTC | TNTC | TNTC | TNTC | TNTC |

| 6 Hours A0.3 *Escherichia coil* | | | | | | |
|---|---|---|---|---|---|---|
| **Amount Plated** | **Dilution Factor** | **Replicate 1** | **Replicate 2** | **Replicate 3** | **Mean** | **Actual CFU/Test Article** |
| **0.1 mL** | **1000** | TNTC | TNTC | TNTC | TNTC | TNTG |
| **1.0 mL** | **100** | TNTC | TNTC | TNTC | TNTC | TNTC |
| **10.0 mL** | **10** | TNTC | TNTC | TNTC | TNTC | TNTC |

| **6 Hours Control 2 *Escherichia coli*** | | | | | | |
|---|---|---|---|---|---|---|
| **Amount Plated** | **Dilution Factor** | **Replicate 1** | **Replicate 2** | **Replicate 3** | **Mean** | **Actual CFU/Test Article** |
| **0.1 mL** | **1000** | 6 | 11 | 9 | 8.67 | 8.7 x 10³ |
| **1.0 mL** | **100** | 41 | 53 | 42 | 45.3 | 4.5 x 10³ |
| **10.0 mL** | **10** | TNTC | TNTC | TNTC | TNTC | TNTC. |

| **6 Hours A0.3 *Enterococcus faecium*** | | | | | | |
|---|---|---|---|---|---|---|
| **Amount Plated** | **Dilution Factor** | **Replicate 1** | **Replicate 2** | **Replicate 3** | **Mean** | **Actual CFU/Test Article** |
| **0.1 mL** | **1000** | 78 | 42 | 138 | 86 | 8.6 x 10⁴ |
| **1.0 mL** | **100** | TNTC | TNTC | TNTC | TNTC | TNTC |
| **10.0 mL.** | **10** | TNTC . | TNTC | TNTC | TNTC | TNTC |

**TABLE IIIA**

| **Log Reduction Values for *Staphylococcus epidermidis*** | | | | |
|---|---|---|---|---|
| | **Sample** | **Actual CFU** | **Log10** | **Log Reduction** |
| | **Inoculum** | 2.6 x 10⁴ | 4.15 | N/A |
| | **A0.3** | 5.1 x 10³ | 3.71 | 0.44 |
| | **Control** 2 | 6.9 x 10³ | 3.84 | 0.31 |

| **Log Reduction Values for *Escherichia coli*** | | | | |
|---|---|---|---|---|
| | **Sample** | **Actual CFU** | **Log10** | **Log Reduction** |
| | **Inoculum** | 7.5 x 10⁵ | 5.88 | N/A |
| | **A0.3** | TNTC | N/A | N/A |
| | **Control 2** | 4.5 x 10³ | 3.65 , | 2.23 |

| **Log Reduction Values for *Enterococcus faecium*** | | | | |
|---|---|---|---|---|
| | **Sample** | **Actual CFU** | **Log10** | **Log Reduction** |
| | **Inoculum** | 1.3 x 10⁵ | 6.11 | N/A |
| | **A0.3** | 8.6 x 10⁴ | 4.93 | 1.18 |
| | **Control** 2 | TNTC | N/A | N/A |

| **Log Reduction Values for *Streptococcus pyrogenes*** | | | | |
|---|---|---|---|---|
| | Sample | Actual CFU | Log10 | Log Reduction |
| | **Inoculum** | 3.3 x 10⁹ | 5.52 | N/A |
| | **A0.3** | 1.1 x 10⁴ | 4.04 | 1.48 |
| | **Control 2** | 7.1 x 10³ | 3.85 | 1.67 |

| **Log Reduction Values for *Staphylococcus aureus*** | | | | |
|---|---|---|---|---|
| | **Sample** | **Actual CFU** | **Log10** | **Log Reduction** |
| | **Inoculum** | . 3 x 10⁵ | 5.48 | N/A |
| | **A0.3** | 8.8 x 10³ | 3.94 | 1.54 |
| | **Control 2** | 1.3 x 10⁶ | 5.11 | 0.37 |

**TABLE IIIB**

| **6 hours *E, faecium A0.3*** | | | | | | |
|---|---|---|---|---|---|---|
| **Amount plated** | **Dilution Factor** | **Replicate 1** | **Replicate 2** | **Replicate 3** | **Mean** | **Actual CPU/Test Article** |
| 0.01mL | 10⁴ | 43 | 39 | 30 | 37 | 3.7 x 10⁶ |
| 0.1 mL | 10³ | TNTC | TNTC | TNTC | TNTC | TNTC |
| 1.0 mL | 10² | TNTC | TNTC | TNTC | TNTC | TNTC |
| 10.0 mL | 10 | TNTC | TNTC | TNTC | TNTC | TNTC |

| **6 hours *E*. *faecium* Control 2** | | | | | | |
|---|---|---|---|---|---|---|
| **Amount plated** | **Dilution Factor** | **Replicate 1** | **Replicate 2** | **Repricate 3** | **Mean** | **Actual CFU/Test Article** |
| 0,01 mL | 10⁴ | 24 | 55 | 63 | 47 | 4,7 x 10⁵ |
| 0.1 mL | 10³ | TNTC | TNTC | TNTC | TNTC | TNTC |
| 1.0 mL | 10² | TNTC | TMTC | TNTC | TNTC | INTC |
| 10.0 mL | 10 | TNTC | TNTC | TNTC | TNTC | TNTC |

| **6 hours *E*. coli A0.3** | | | | | | |
|---|---|---|---|---|---|---|
| **Amount plated** | **Dilution Factor** | **Replicate 1** | **Replicate 2** | **Replicate 3** | **Mean** | **Actual CFU/Test Article** |
| 0.01 mL | 10⁴ | 8 | 2 | NOC | 3 | 3.0 x 10⁴ |
| 0.1 mL | 10⁴ | 29 | 60 | 93 | 61 | 6.1 x 10⁴ |
| 1.0 mL | 10² | 159 | TNTC | TNTC | TNTC | TNTC |
| 10.0 mL | 10 | TNTC | TNTC | TNTC | TNTC | TNTC |

| **6 hours *E*. *coli control 2*** | | | | | | |
|---|---|---|---|---|---|---|
| **Amount plated** | **Dilution Factor** | **Replicate 1** | **Replicate 2** | **Replicate 3** | **Mean** | **Actual CFU/Test Article** |
| 0.01 mL | 10⁴ | 57 | 40 | 60 | 62 | 62 x 10⁴ |
| 0.1 mL | 10³ | TNTC | TNTC | 153 | TNTC | TNTC |
| 1.0 mL | 10² | TNTC | TNTC | TNTC | TNTC | TNTC |
| 10.0 mL | 10 | TNTC | TNTC | TNTC | TNTC | TNTC |

| **6 hours *C*. *albicans A0.3*** | | | | | | |
|---|---|---|---|---|---|---|
| **Amount plated** | **Dilution Factor** | **Replicate** | **1 Replicate 2** | **Replicate 3** | **Mean** | **Actual CPU/Test Article** |
| 0,01 mL | 10⁴ | NOC | NOC | NOC | NOC | NOC |
| 0.1 mL | 10³ | NOC | NOC | NOC | NOC | NOC |
| 1.0 mL | 10² | NOC | NOC | 7 | 2 | 2.0 x 10² |
| 10.0 mL | 10 | 33 | 37 | 65 | 46 | 4.5 x 10² |

| **6 hours *C, albicans Control 2*** | | | | | | |
|---|---|---|---|---|---|---|
| **Amount plated** | **Dilution Factor** | **Replicate 1** | **Replicate 2** | **Replicate 3** | **Mean** | **Actual CPU/Test Article** |
| 0.01 mL | 10⁴ | NOC | NOC | NOC | NOC | NOC |
| 0.1 mL | 10³ | NOC | NOC | NOC | NOC | NOC |
| 1.0 mL | 10² | NOC | 2 | 1 | 3 . | 3.0 x 10² |
| 10.0 mL | 10 | 36 | 49 | 53 | 46 | 4.6 x 10² |

**TABLE IIIC**

| **Log Reduction Values *E. faecium* Test Article and Control (0.01 mL Recovery)** | | | |
|---|---|---|---|
| **Sample** | **Actual CFU/Tast Article** | **Log₁₀** | **Log Reduction** |
| Inoculum | 2.8 x 10⁶ | 6.45 | N/A |
| A0.3 | 3.7 x 10⁵ | 5.57 | 0.88 |
| Control 2 | 4.7 x 10⁵ | 5.67 | 0.78 |

| **Log Reduction Values *E*. *coli* Test Article and Control (0.1 mL Recovery)** | | | |
|---|---|---|---|
| **Sample** | **Actual CFU/Test Article** | **Log₁₀** | **Log Reduction** |
| Inoculum | 1.2 x 10⁶ | 6.08 | N/A |
| A0.3 | 6.1 x 10⁴ | 4.79 | 1.29 |
| Control 2 | 5.2 x 10⁵ | 5.72 | 0.36 |

| **Log Reduction Values *C. albicans* Test Article and Control (10 mL Recovery)** | | | |
|---|---|---|---|
| **Sample** | **Actual CFU/Test Article** | **Log₁₀** | **Log Reduction** |
| Inoculum | 2.0 x 10⁶ | 6.30 | N/A |
| A0.3 | 4.5 x 10² | 2.65 | 3.65 |
| Control 2 | 4.6 x 10² | 2.66 | 3.64 |

Log reduction effectiveness testing was conducted on two occasions, Tables IIA-IIC, and Tables IIA & IIIB being associated with results for the separate test occasions. Log reduction of inoculated antimicrobial materials, more particularly the protocol associated therewith, identifies the time log reduction of inoculated organisms on test article surfaces. Five different test organisms were tested with the subject solvent based antimicrobial adhesive system as applied to an incised film, and a positive control, namely, the heretofore described and disclosed solventless hot melt as applied to an incise film.

The first study, (i. e. , that relating to Tables II), indicate that the subject solvent based antimicrobial adhesive system was at least equivalent for three of the organisms tested. However, E. coli and E. faecium required the use a different dilution factor for the inoculum. In the second, later study (i. e., that associated with Tables III), the subject organisms were tested at a higher dilution factor (0.01 ml), as was the fungus C. albicans. The results indicate that the subject solvent based antimicrobial adhesive is at least equivalent to the solventless hot melt previously described herein.

New characteristics and advantages of the invention covered by this document have been set forth in the foregoing description. It will be understood, however, that this disclosure is, in many respects, only illustrative. Changes may be made in details, particularly in matters of shape, size, and arrangement of parts, without exceeding the scope of the invention. The scope of the invention is, of course, defined in the language in which the appended claims are expressed.

## Claims

1. A solvent based adhesive system having antimicrobial properties for skin contact applications comprising
a solid powder antimicrobial agent;
a solvent-based acrylic polymer adhesive, and
a substrate,
said antimicrobial agent being directly compounded into said solvent-based acrylic adhesive so as to define an antimicrobial adhesive composition,
wherein said antimicrobial agent is an effective amount of diiodomethyl-p-tolylsufone dispersed throughout said acrylic polymer.

2. The system of claim 1, wherein said acrylic polymer comprises a mixture of acrylic resin solutions.

3. The system of claim 2, wherein said mixture of acrylic resin solutions includes a non self-curing acrylic resin solution.

4. The system of claim 3, wherein said mixture of acrylic resin solutions includes a self-curing acrylic resin solution.

5. The system of claim 4, wherein a concentration of diiodomethyl-p-tolylsulfone in said acrylic polymer is between 0.1 % by weight to 2% by weight, preferably between 0.1% by weight to 1% by weight, and most preferably between 0.1% by weight to 0. 5% by weight.

6. The system of claim 5, wherein said non self-curing acrylic resin solution comprises a majority of said mixture of acrylic resin solutions.

7. The system of claim 6, wherein a weight based ratio of said non self-curing acrylic resin solution to said self-curing resin solution for said mixture of acrylic resin solutions is between 7 and 10.

8. The system of claim 7, wherein said non self-curing acrylic resin solution of said mixture of acrylic resin solutions comprises 40 % by weight to 45 % by weight solids.

9. The system of claim 8, wherein said self-curing acrylic resin solution of said mixture of acrylic resin solutions comprises 30 % by weight to 35 % by weight solids.

10. A surgical drape comprising a sheet of polymeric substrate and a coating of an adhesive composition according to claim 1, overlying said polymeric substrate.

## Patentansprüche

1. Auf Lösungsmittel basierendes Klebesystem mit antimikrobiellen Eigenschaften für Hautkontaktanwendungen umfassend:
ein antimikrobielles Festpulvermittel;
ein auf Lösungsmittel basierendes, Acrylpolymer-Klebemittel, und
ein Substrat,
wobei das antimikrobielle Mittel direkt in das auf Lösungsmittel basierende Acrylklebemittel eingearbeitet ist, um eine antimikrobielle Klebstoffzusammensetzung zu definieren,
wobei das antimikrobielle Mittel eine wirksame Menge von Diiodomethyl-p-tolylsulfon ist, welches in dem gesamten Acrylpolymer dispergiert ist.

2. Das System nach Anspruch 1, wobei das Acrylpolymer eine Mischung aus Acrylharzlösungen aufweist.

3. Das System nach Anspruch 2, worin die Mischung aus Acrylharzlösungen eine nicht selbstaushärtende Acrylharzlösung umfasst.

4. Das System nach Anspruch 3, worin die Mischung aus Acrylharzlösungen eine selbstaushärtende Acrylharzlösung umfasst.

5. Das System nach Anspruch 4, worin die Konzentration von Diiodomethyl-p-tolylsulfon in dem Acrylpolymer zwischen 0,1 Gew.-% bis 2 Gew.-%, vorzugsweise zwischen 0,1 Gew.-% bis 1 Gew.-% und am meisten bevorzugt zwischen 0,1 Gew.-% bis 0,5 Gew.-% beträgt.

6. Das System nach Anspruch 5, worin die nicht selbstaushärtende Acrylharzlösung eine Mehrheitskomponente der Mischung aus den Acrylharzlösungen umfasst.

7. Das System nach Anspruch 6, worin ein auf Gewicht basierendes Verhältnis der nicht selbstaushärtenden Acrylharzlösung zu der selbstaushärtenden Harzlösung für die Mischung aus Acrylharzlösungen zwischen 7 und 10 liegt.

8. Das System nach Anspruch 7, worin die nicht selbstaushärtende Acrylharzlösung und die Mischung aus Acrylharzlösungen 40 Gew.-% bis 45 Gew.-% umfassen.

9. Das System nach Anspruch 8, worin die selbstaushärtende Acrylharzlösung der Mischung aus Acrylharzlösungen 30 Gew.-% bis 35 Gew.-% umfasst.

10. Ein chirurgisches Verbandmaterial umfassend eine Schicht aus Polymersubstrat und eine Beschichtung aus einer Klebstoffzusammensetzung gemäß Anspruch 1, die über dem Polymersubstrat liegt.

## Revendications

1. Système adhésif à base de solvant ayant des propriétés antimicrobiennes pour les applications en contact avec la peau comprenant
un agent antimicrobien de poudre solide;
un adhésif de polymère acrylique à base de solvant, et
un substrat,
ledit agent antimicrobien étant directement composé dans ledit adhésif acrylique à base de solvant pour définir une composition adhésive antimicrobienne,
où ledit agent antimicrobien est une quantité efficace de diioddométhyle-p-tolylsulfone dispersée partout dans ledit polymère acrylique.

2. Système selon la revendication 1, où ledit polymère acrylique comprend un mélange de solutions de résine acrylique.

3. Système selon la revendication 2, où ledit mélange de solutions de résine acrylique comprend une solution non auto-durcissante de résine acrylique.

4. Système selon la revendication 3, où ledit mélange de solutions de résine acrylique comprend une solution auto- durcissante de résine acrylique.

5. Système selon la revendication 4, où une concentration de diioddométhyle-p-tolylsulfone dans ledit polymère acrylique est entre 0,1% en poids et 2% en poids, préférablement entre 0,1 % en poids et 1% en poids, et le plus préférablement entre 0,1 % en poids et 0,5% en poids.

6. Système selon la revendication 5, où ladite solution non auto- durcissante de résine acrylique comprend la plupart dudit mélange de solutions de résine acrylique.

7. Système selon la revendication 6, où un rapport des poids de ladite solution non auto-durcissante de résine acrylique à ladite solution auto- durcissante de résine acrylique pour ledit mélange de solutions de résine acrylique est entre 7 et 10.

8. Système selon la revendication 7, où ladite solution non auto- durcissante de résine acrylique dudit mélange de solutions de résine acrylique comprend 40% en poids à 45% en poids de matières solides.

9. Système selon la revendication 8, où ladite solution auto- durcissante de résine acrylique dudit mélange de solutions de résine acrylique comprend 30% en poids à 35% en poids de matières solides.

10. Un drap chirurgical comprenant une feuille de substrat de polymère et un revêtement d'une composition adhésive selon la revendication 1 recouvrant ledit substrat de polymère.
